Europäisches Patentamt

(19) European Patent Office    (11) Publication number: **0 011 511**

Office européen des brevets    **B1**

(12)    **EUROPEAN PATENT SPECIFICATION**

(45) Date of publication of patent specification: **07.11.84**    (51) Int. Cl.³: **A 61 K 7/09**

(21) Application number: **79302642.8**

(22) Date of filing: **20.11.79**

(54) **A method and compositions for relaxing natural curls and kinks in human hair and a package for these compositions.**

(30) Priority: **20.11.78 US 961917**

(43) Date of publication of application:
**28.05.80 Bulletin 80/11**

(45) Publication of the grant of the patent:
**07.11.84 Bulletin 84/45**

(84) Designated Contracting States:
**BE DE FR GB IT**

(56) References cited:
**DE-A-1 492 176**
**DE-A-1 802 412**
**DE-A-2 260 049**
**GB-A- 773 559**
**GB-A-1 198 857**
**US-A-2 850 351**
**US-A-3 227 615**

The file contains technical information
submitted after the application was filed and
not included in this specification

(73) Proprietor: **Lamaur Inc.**
**5601 East River Road**
**Minneapolis, Minnesota 55440 (US)**

(72) Inventor: **Moore, Edward R.**
**R.R. No. 2. Box 241**
**Princeton, Minnesota 55371 (US)**
Inventor: **Riddle, Eldred O.**
**7865 Tessman Drive**
**Brooklyn Park, Minnesota 55445 (US)**

(74) Representative: **Sanderson, Laurence Andrew**
**et al**
**Sanderson & Co. 97 High Street**
**Colchester Essex C01 1TH (GB)**

**0011511**

## Description

This invention relates to a method for relaxing natural curls and kinks in human hair by treatment thereof with a curl-relaxing composition while manipulating the treated hair into a relatively-straightened condition, and a package containing a combination of compositions suitable for use in said method.

Natural kinks and curls in human hair are an inherited trait, attributable to irregular bonding of internal portions of the hair. If it is desired to impart any chosen style to kinky or curly hair, usually it will be necessary first to straighten such hair at least to some extent. This straightening procedure does not necessarily result in perfectly straight hair, but does bring it to at least a relatively smooth S-curved condition — and is commonly referred to as "relaxing" the hair. It can be accomplished, to a greater or lesser extent, by first disrupting the internal bonds of the hair, then manipulating and extending the hair to achieve some degree of straightness, and thereafter trying to rebuild the internal bonds of the hair so as to prevent it from reverting ultimately to its original kinky or curly state.

One of the known treatments currently employed to relax hair involves the use of ammonium thioglycolate, commonly used in the past for producing a "cold permanent wave". Unfortunately however the relaxing effect achieved using ammonium thioglycolate is often insufficient, particularly on kinky hair; and even when adequate relaxation is initially achieved, it has been found that there is generally-speaking quite a probability that the hair will ultimately revert to its original curly or kinky condition.

Because it is more effective the most popular treatment currently employed to relax the hair while it is still growing from the head involves the use of so-called "lye", that is to say a strongly-alkaline solution of sodium hydroxide. Although this is quite effective it however has significant disadvantages. When employing sodium hydroxide (or lye) there is considerable danger of damage to both the hair and the scalp. Re-treatment of previously-treated hair may result in breakage of the hair-shaft at the junction between the new growth and the previously-treated hair. Moreover great care must be exercised before applying the treatment, to make quite sure that there are no abrasions or sores on the scalp, since if the sodium hydroxide is able to enter below the skin it will cause burns; and it is in fact conventional, in order to help protect the skin, to apply a base such as petroleum jelly to the scalp before the treatment with the sodium hydroxide solution is commenced.

So far as we are aware no equally effective and practical alternative to the above-mentioned strongly-alkaline solutions has hitherto been discovered, and certainly none has been commercialized. We however have now found a sequence of treatments which involves the use of a weakly acidic curl-relaxing solution which thus is relatively safe and painless, and yet is a very effective treatment for human hair capable of straightening out natural kinks and curls therein, and thereafter fixing the hair in this relatively-straightened condition. Our treatment sequence is moreover able simultaneously to preserve or impart the desired "liveliness", "buoyancy" and "managability" in the air. It therefore represents a breakthrough in the treatment of curly hair, which indeed is the more astonishing when it is realized that all of the significant chemicals employed in our treatment sequence have been available for several decades, even in the hands of those who market the current commercial strongly-alkaline treatments.

According to this invention there is provided a method for relaxing natural curls and kinks in human hair, by treatment thereof (including the root scalp portions thereof) with a curl-relaxing composition while manipulating the thus-treated hair by hand-action and/or combing into a relatively-straightened condition and subsequently rinsing the hair, characterized in that

(1) the curl-relaxing composition employed is a weakly-acidic aqueous solution, having a pH value of less than 7 but greater than 5, of a salt of an acid capable of reducing the disulphide linkages of the keratin in hair;

(2) the thus-treated hair is then heated to a temperature above human body temperature, while simultaneously maintaining the moisture content of the heated hair substantially constant, until it exhibits a relaxed condition;

(3) the scalp and the thus-relaxed hair are then water-rinsed to remove the curl-relaxing composition therefrom, and thereafter the rinsing water is at least partially removed from the relaxed hair to achieve an at least towel-blotted state;

(4) the rinsed hair is then treated with a fixative composition comprising an aqueous solution, having a pH not greater than 9, of a weakly alkaline substance capable of chemically neutralizing the curl-relaxing composition;

(5) the neutralized hair is then water-rinsed to remove the fixative composition therefrom, and thereafter the rinsing water is at least partially removed therefrom to achieve an at least towel-blotted state; and

(6) the hair finally is treated to stabilize its condition by applying thereto a bonding composition comprising an aqueous solution of a thermo-setting aminopolyamide resin.

The sequence of treatments according to the present invention overcomes the problems encountered with previously-available treatments. Relatively speaking our treatment sequence seems

2

to involve little or no danger for the user, either to his hair or his scalp. Hair previously-straightened by our treatment sequence can be re-treated by the same technique, even immediately subsequent to the prior treatment, without any apparent damage to the hair. Moreover our treatment sequence seems to cause no significant skin or scalp irritation or other pain or discomfort to anyone with a normal healthy, scalp condition. Furthermore, in contrast to the previously-conventional application of a protective coating (such as petroleum jelly) it is possible before our treatment sequence for the person undergoing the treatment to be given a shampoo, so as thoroughly to cleanse the hair and the scalp.

Before carrying out the method of this invention it is very desirable to ensure that the hair and scalp are completely clean. Then to the clean hair there is applied a curl-relaxing composition which essentially must contain an aqueous solution of a salt of an acid capable of reducing the cystine-linkages of hair keratin, and having a pH of less than 7 but more than 5. There are a number of such salts, as will be known to those skilled in this art, but pre-eminent amongst them is ammonium bisulphite. The preferred curl-relaxing composition will thus contain an aqueous solution of ammonium bisulphite buffered to a pH within the range of 5—7.

While we do not wish to be limited by any theoretical considerations it is believed that the ammonium bisulphite severs the disulphide-linkages (KSSK) in keratin; and is also thought to convert one of the sulphurs of the cystine-linkage into a sulphydryl group (KSH), while forming a Bunte salt $(KSSO_3^- + NH_4^+)$ with the other sulphur of the cystine-linkage.

The curl-relaxant composition should be applied so that it saturates the hair. This can be accomplished by applying the aqueous composition direct to the hair and then working it thoroughly into the hair by hand and/or by repeated combing which helps to extend the hair. Alternatively it may also be accomplished by applying the aqueous composition to a fabric or spongy material arranged to overlie the hair and which thus will tend to saturate it with the composition exuding therefrom. Application of the aqueous composition and subsequent manipulation of the hair is facilitated if the composition includes a gel-forming material which will create a gel-like composition. Such gel-like compositions not only facilitate application by causing the composition to adhere to the hair, but also have the added advantage of lending "body" to the hair, which aids in the subsequent straightening operation. The gel-forming material in the composition functions as an inert bonding material for holding strands of curly hair in a somewhat stretched and straightened condition against each other and against the scalp.

The gel-forming material used to impart a gel-like consistency to the aqueous composition may be any suitable inert thickener, preferably an inert organic thickener such as hydroxy ethyl cellulose. Despite the presence of the gel-forming material it is however desirable that the water-content of the composition should exceed 50% of the total bulk of the gel; and for convenience in application the inert thickener should preferably be present in such an amount as to cause the viscosity of the gel to lie between 80 and 400 Newton seconds per square metre at 25°C. Other useful known ingredients may optionally be included to serve their usual function. Whatever gel-forming materials and other ingredients may be present the pH of the aqueous curl-relaxing composition must lie within the weakly-acidic range of 5 to 7, and preferably will not be below about 5.7.

Following application of the curl-relaxing composition thereto the hair must be heated above human body temperature (thus above about 38°C) while maintaining its water-content substantially constant. This can be readily achieved by enclosing the hair in a moisture-impervious cap, and then subjecting it to mild heating. This step is of fundamental importance to the success of the method of this invention, presumably because heat accelerates the chemical reaction between the curl-relaxing composition and the hair. The degree and duration of the heating to be employed will of course depend upon the degree of curliness and/or the toughness and resilience of the hair. For general guidance it can however be said that one should aim to create a temperature of from 38°C to 50°C and to maintain it for from 5 to 20 minutes.

After heating has ceased it is of considerable importance to smooth and straighten the hair with comb and hands while the hair is still warm. The smoothing action should start at the roots of the hair, using the thumbs and the flat back portion of the comb; and the comb should have its tines turned upward to create some tension on the hair. This procedure should be followed until it can be seen whether or not the proper degree of straightening has been attained. If not, the water-impermeable plastic cap can be put back over the head, and the heating continued for a further period — since this process is not harmful to the hair. There is indeed a considerable latitude in the application of the method of this invention to the relaxing of kinky or curly hair, and the temperature and duration of the heat treatment and the severity of the manipulation of the hair can all be varied quite widely, depending upon the degree of curliness of the hair, which will seldom be the same for any two people.

In a preferred procedure, the curl-relaxing composition is formulated as a gel, which is thoroughly worked into the hair by means of hand moulding and combing the hair into a stretched, relatively-straight condition. Although the gel-forming material is inert chemically, it co-operates in effecting the straightening of the hair by adding body to the hair.

Before proceeding to the next stage in the treatment the curl-relaxing composition should of course be very thoroughly rinsed from the hair and scalp.

Following the curl-relaxing treatment the relaxed hair keratin is then subjected to a fixative treatment, which involves applying and working into the hair an aqueous solution, having a pH not greater than about 9, of a weakly-alkaline substance which is able chemically to neutralize the curl-relaxing composition. As in the case of the curl-relaxing composition, this fixative composition must also be applied in such a manner as to saturate the hair, which may be accomplished by procedures essentially similar to those used and described above with the curl-relaxing composition. In an analogous manner the fixative composition may also advantageously incorporate an inert thickener to impart a gel-like consistency thereto, thereby simplifying application to the hair.

The aqueous solution used in this fixative treatment must contain water to serve as a vehicle for ionization of the weakly-alkaline substance, which preferably will be the salt of a weak acid. Examples of such salts of weak acids, exhibiting an alkaline pH below about 9, are sodium bicarbonate (i.e. baking soda), ammonium bicarbonate, disodium phosphate and the like. They contain oxygen in their anion; and the acid hydrogen present is weaker than the alkaline or base strength of the basic radical or cation.

While we do not wish to be limited by any theoretical considerations it appears that the function of the weakly-alkaline substance, or specifically of the weak acid salt, is to react with a Bunte salt complex formed at some of the sulphurs of the several cystine-linkages in the hair keratin during the curl-relaxation step. The assumed reaction is complex, but (aside from by-products later rinsed out of the hair) is believed to cause the Bunte salt complexes to be converted to sulphydryl groups (KSH), thereby increasing the number of sulphydryl groups beyond that attainable with the above-described curl-relaxing treatment alone. At all events, the fixative treatment does at least contribute to further alteration of the severed disulphide-linkages of the hair keratin; and the formation of additional sulphydryls is the most plausible explanation from an empirical standpoint. Whatever the explanation, this fixative treatment contributes to the re-orientation of the hair keratin toward a relaxed, straightened condition for the strands of hair, and additionally obstructs re-connection of several cystine-linkages back to their original condition.

It is advantageous if the aqueous fixative composition, like the previously-described curl-relaxing composition, contains a gel-former such as an organic thickener. In this fixative treatment the function of the gel-former is that of co-operatively working with the weakly-alkaline substance for enhancing the straightened condition for the hair strands.

Before proceeding to the next stage in the treatment the fixative composition should of course be very thoroughly rinsed from the hair and scalp.

Following the fixative treatment, the hair finally is treated to stabilize it in its relaxed condition. Stabilization is accomplished by the application of a stabilizing composition (or bonding lotion) containing a thermosetting aminopolyamide resin, preferably a cationic water-soluble thermosetting aminopolyamide-epichlorohydrin resin — which is able to alter the hair keratin sulphydryls, and thus prevent subsequent reconnection of the cystine-linkages which had been severed by the curl-relaxing composition.

The term "aminopolyamide-epichlorohydrin resin" as used herein refers to the water-soluble polymeric reaction product of epichlorohydrin and a polyamide derived from a poly-alkylene polyamine and a $C_3$—$C_7$ saturated aliphatic dicarboxylic acid. Such resins and their preparation have been described in United States Patent No. 3,227,615. Briefly stated, the preparation of such resins involves first reacting one or more of the dicarboxylic acids with one or more of the polyalkylene polyamines, under such conditions as will produce a water-soluble polyamide containing the recurring groups —$NH(C_nH_{2n}HN)x$—CORCO— (where $n$ and $x$ are each 2 or more, and R is the divalent hydrocarbon radical of the dicarboxylic acid) and then reacting the resultant water-soluble polyamide with epichlorohydrin to form the desired water-soluble, cationic, thermosetting resin. In the second of these stages the epichlorohydrin reacts with the secondary amines of the water-soluble polyamide, to form azetidinium functional groups or equivalent groups; and the latter are in turn able to react with the sulphydryl formed in the hair keratin.

The large number of sulphydryl sites created in the hair keratin by the curl-relaxing treatment of this invention makes possible a massive incorporation of the complex polyamide-epichlorohydrin resin into the complex keratin of the hair, thus giving the hair "body" and "manageability", as well as almost totally preventing it from reversion to its original curly state. By means of the stabilizing treatment the relaxed hair is, for practical purposes, retained permanently in the straightened condition which the subject desires. This straightened condition will generally approximate to an open or loose S-curve in each of the strands of hair, which is easier to manage than naturally curly or kinky hair and may as such satisfy the subject. Should the subject desire the hair thus straightened can however then be further styled or even curled to various degrees in different parts in whatever manner will satisfy the desires of individual taste.

Hair which has been treated according to the method of this invention may indeed be set using any conventional procedures. The treatment sequence of this invention will indeed smooth out and tend to straighten all types of naturally curly hair, and it is safe to repeat treatments as new growth occurs.

The compositions for use in the method of the invention are advantageously provided by a package suitable for use in the method, in which package the present curl-relaxing composition, the present fixative composition and the present stabilizing composition are each separately present but arranged for use sequentially in said method.

Such a package may very desirably also include a water-impermeable plastic cap for use in conjunction with the curl-relaxing composition during the heat-treatment stage of the method.

In order that the invention may be well understood it will now be described in more detail, though only by way of illustration, in the following Examples:

Example I
Preparation of compositions A, B and C
Composition A: relaxer gel formulation

A curl-relaxing composition in the form of a gel was formulated from the ingredients listed below, in the stated amounts (the percentages being by weight), as follows:—

| | |
|---|---|
| Deionized water | 55.55% |
| Aqueous ammonium bisulphite (47% concentration) | 22.00% |
| Hydroxy ethyl cellulose | 2.50% |
| Urea | 10.00% |
| Isopropanol, 99% anhydrous | 5.00% |
| Dibasic sodium phosphate | 1.14% |
| Citric acid | 0.46% |
| Ammonium hydroxide | 1.10% |
| Chelating agent | 0.05% |
| Organic dye | 0.0001% |
| Perfume | 0.20% |
| Non-ionic surfactant | 2.00% |

Notes
(a) The hydroxy ethyl cellulose serves as an inert water-soluble organic thickener;
(b) The urea breaks down on heating and helps to buffer the pH to 6.2, while it also causes or contributes to the swelling of the hair strands which assist the action of the ammonium bisulphite;
(c) The *iso*propanol helps in wetting the hair fibres;
(d) The dibasic sodium phosphate contributes hydrogen ion and influences the pH;
(e) The citric acid and ammonium hydroxide assist in buffering the pH;
(f) The organic dye is included merely to tint the composition to an appropriate colour; and
(g) The non-ionic surfactant is present merely to solubilize the perfume.

The above-listed ingredients were mixed together and blended into a homogeneous mass by dissolving and thoroughly mixing the chelating agent, sodium phosphate, citric acid, urea, and hydroxy ethyl cellulose into the water. In order to facilitate solubilization of the cellulose thickener the mixture was heated to about 45°C. After re-cooling to about 25°C, the organic dye dissolved in *iso*propanol was added and thoroughly mixed in, followed by a blend of the ammonium bisulphite and ammonium hydroxide, which again were thoroughly mixed. Finally the perfume blended with the non-ionic surfactant was added and thoroughly mixed in so as to blend all the ingredients into a homogeneous mass.

Composition B: fixative gel formulation
A fixative composition in the form of a gel was formulated from the ingredients listed below, in the stated amounts (the percentages being by weight), as follows:

| Deionized water | 89.55% |
|---|---|
| Sodium bicarbonate | 5.00% |
| Hydroxy ethyl cellulose | 2.50% |
| Sodium hydroxide | 0.05% |
| Chelating agent (trisodium hydroxy-ethyl ethylenediamine-triacetate) | 0.20% |
| Non-ionic surfactant (polyethylene [23] lauryl ether) | 2.00% |
| Preservatives, including bactericides and fungicides, namely methyl parasept, propyl parasept, umidazolidinyl urea) | 0.70% |

Notes
(a) The sodium bicarbonate seves as the weak acid salt;
(b) The hydroxy ethyl cellulose serves as the inert water-soluble thickener;
(c) The sodium hydroxide serves for pH adjustment;
(d) The chelating agent serves to counteract metal impurities; and
(e) The non-ionic surfactant serves as solubilizer for the preservatives.

The above-listed ingredients were mixed together and blended into a homogeneous mass by dissolving the sodium bicarbonate in the water, then adding the other ingredients, with continuous mixing while heating to 55°—60°C until homogeneity is achieved, after which it is cooled to ambient temperature.

The resultant composition is buffered to a pH of about 8.2. Its viscosity at 25°C is about 250 Newton seconds per square metre.

Composition C: stabilizing formulation
A stabilizing composition was formulated from the ingredients listed below, in the stated amounts (the percentages being by weight), as follows:

| Deionized water | 95.60% |
|---|---|
| Low molecular weight resin form of adipic acid-diethylene-triamine polyamide reacted with epichlorohydrin and secondary amines to give azetidinium functional groups which react with sulphydrul | 2.00% |
| Quaternized vinylpyrrolidone copolymer | 2.00% |
| Non-ionic surfactant (polyoxyethylene [9] octylphenyl ether) | 0.30% |
| Perfume or other fragrance | 0.05% |
| Preservative, namely formalin | 0.05% |

Notes
(a) The low molecular weight resin used was that marketed under the name "DELSETTE" (assumed to be a registered trade mark) by Hercules; and
(b) The quaternized vinylpyrrolidone copolymer is optional but contributes to hair-manageability.

The above-listed ingredients were simply mixed together at ambient temperature to form a bonding lotion.

Example II
Treatment sequence for curl-relaxation pretreatment stage — cleansing the hair and scalp
In contrast with the conventional procedure involving treatment with lye, for the treatment sequence of this invention the hair and scalp should preferably be clean and free from all forms of foreign matter, thus not only grease and other protective coatings but also any normal soiling-matter such as atmospheric dust, dried perspiration and any form of previously-used hair-care composition. To achieve that condition, the hair and scalp should be properly shampooed, thoroughly rinsed and towel-dried, so that the subsequent treatment described below will be equally effective upon all of the hair upon the scalp.

**0011511**

Curl-relaxing stage 1

After preliminary cleaning in the manner described above, the hair is prepared to receiving the curl-relaxing composition by parting the hair from front to back and from ear to ear, thus creating four sections. A curl-relaxing composition such as Composition A described in Example I above is then applied to the root-areas of the hair, beginning with the back sections and advancing towards the crown, and then going on to the front sections, but leaving the hairline until last. The curl-relaxing composition is applied to the hair by working outwardly from the root portions, and then worked thoroughly throughout the hair strands by hand moulding and by combing the hair out into a stretched, relatively-straight condition, with the hair pasted against the scalp. If the hair is long, it should be folded and placed on top of the head.

Heat-treatment stage 2

The hair thus moulded to the head and thoroughly saturated with Composition A is then subjected to a heat treatment while maintaining the moisture content of the composition in the hair substantially constant by covering the head with a flexible, water-impermeable plastic cap (formed of polyethylene or any similar plastic material) so that it serves as a barrier against the escape of water. The hair thus covered is then heated by surrounding the covered head with a conventional heating hair-dryer, capable of heating the hair above 38°C, and preferably up to about 50°C. Heat is applied in this manner for a period between 5 and 20 minutes, the optimum time being determined empirically by periodical removal of the plastic cap, and examination or testing of a strand of hair to determine the effect of the curl-relaxing composition.

When the results of this examination (determined visually) or other testing seem acceptable, the flexible plastic head-covering is removed, and the still-warm hair still saturated with the curl-relaxing composition therein is further worked by hand — smoothing it out, combing through the hair from the root areas, and finally turning the comb to create tension in the hair. This hand-working and combing operation should be carried on for about 20 minutes, or if necessary longer until the hair is relaxed to the desired extent.

A visual test for the extent of curl relaxation is as follows. If a portion of the strands of hair adjacent the scalp remains against the scalp, then generally-speaking the hair will have become sufficiently relaxed. A useful additional test is to rest a strand of hair, which has been wiped free from composition using a wet towel, upon the palm of one's hand thus to observe its degree of relaxation before terminating this post-heating treatment.

Water-rinsing stage 3

The hair and scalp are thoroughly rinsed with tepid water, and then towel-blotted to achieve a damp condition. Rinsing should be very thorough, and thus generally-speaking should continue for as much as 5 minutes.

Fixative treatment stage 4

A fixative composition such as Composition B described in Example I above is then applied to the hair at normal room temperature, thoroughly worked into the hair (including the root portions) and allowed to remain on the hair for about 5 to 10 minutes.

Water-rinsing stage 5

The fixative composition is then thoroughly rinsed from the hair with tepid water; and the hair is then towel-blotted to a state of semi-dryness.

Stabilizing stage 6

A stabilizing composition such as the bonding lotion Composition C described in Example I above is then thoroughly worked into the hair and is permitted to remain on the hair to aid in setting and stabilizing the hair. This composition, apart from its high water-content, is primarily resinous — but the only essential resin is the aminopolyamide-epichlorohydrin.

**Claims**

1. A method for relaxing natural curls and kinks in human hair, by treatment thereof (including the root scalp portions thereof) with a curl-relaxing composition while manipulating the thus-treated hair by hand-action and/or combing into a relatively-straightened condition and subsequently rinsing the hair, characterized in that

(1) the curl-relaxing composition employed is a weakly-acidic aqueous solution, having a pH value of less than 7 but greater than 5, of a salt of an acid capable of reducing the disulphide linkages of the keratin in hair;

(2) the thus-treated hair is then heated to a temperature above human body temperature, while

7

simultaneously maintaining the moisture content of the heated hair substantially constant, until it exhibits a relaxed condition;

(3) the scalp and the thus-relaxed hair are then water-rinsed to remove the curl-relaxing composition therefrom, and thereafter the rinsing water is at least partially removed from the relaxed hair to achieve an at least towel-blotted state;

(4) the rinsed hair is then treated with a fixative composition comprising an aqueous solution, having a pH not greater than 9, of a weakly alkaline substance capable of chemically neutralizing the curl-relaxing composition;

(5) the neutralized hair is then water-rinsed to remove the fixative composition therefrom, and thereafter the rinsing water is at least partially removed therefrom to achieve an at least towel-blotted state; and

(6) the hair finally is treated to stabilize its condition by applying thereto a stabilizing composition comprising an aqueous solution of a thermosetting aminopolyamide resin.

2. A method as claimed in claim 1, in which the curl-relaxing composition employed contains an inert gel-forming thickener present in an amount such that the water content of the composition exceeds 50% of the total bulk of the gel and the composition has a viscosity between 80 and 400 Newton seconds per square metre at 25°C.

3. A method as claimed in claim 1 or claim 2, in which the pH of the aqueous curl-relaxing composition lies within the weakly-acidic range of from 5.7 to 7.

4. A method as claimed in any of claims 1 to 3, in which the resin in the stabilizing composition is a cationic thermo-setting aminopolyamide-epichlorohydrin resin.

5. A package suitable for use in the method claimed in claim 1, in which package the curl-relaxing composition defined in claim 1, the fixative composition defined in claim 1 and the stabilizing composition defined in claim 1 are each separately present but arranged for use sequentially in said method.

6. A package as claimed in claim 5, which also includes a water-impermeable plastic cap for use in conjunction with the curl-relaxing composition during the heat-treatment stage of the method.

7. A package according to claim 5 or 6 in which the curl-relaxing composition contains an inert gel-forming thickener present in an amount such that the water content of the composition exceeds 50% of the total bulk of the gel and the composition has a viscosity between 80 and 400 Newton seconds per square metre at 25°C.

8. A package according to any one of claims 5—7 in which the pH of the aqueous curl-relaxing composition lies within the weakly-acidic range of from 5.7 to 7.

9. A package according to any one of claims 5—8 in which the resin in the stabilizing composition is a cationic thermo-setting aminopolyamide-epichlorohydrin resin.

**Patentansprüche**

1. Verfahren zum Glätten von naturkrausem und verfitztem menschlichen Haar durch Behandlung des Haares (einschließlich der Kopfhautanteile an der Haarzwurzel) mit einer haarglättenden Zusammensetzung, wobei das so behandelte Haar mit der Hand und/oder durch Kämmen in einen relativ geglätteten Zustand gebracht wird, und anschließendes Spülen des Haares, dadurch gekennzeichnet, daß

(1) die angewandte haarglättende Zusammensetzung aus einer schwach sauren, einen pH-Wert von weniger als 7 und mehr als 5 aufweisenden wäßrigen Lösung eines Salzes einer Säure besteht, die in der Lage ist, die Disulfidbindungen des Haarkeratins zu reduzieren;

(2) das so behandelte Haar anschließend auf eine über der Körpertemperatur liegende Temperatur erwärmt wird, wobei gleichzeitig der Feuchtigkeitsgehalt des erwärmten Haares im wesentlichen konstant gehalten wird, bis das Haar einen geglätteten Zustand aufweist;

(3) die Kopfhaut und das so behandelte Haar dann zur Entfernung der haarglättenden Zusammensetzung mit Wasser gespült werden, worauf das Spülwasser wenigstens teilweise aus dem geglätteten Haar entfernt wird, um einen zumindest handtuchtrockenen Zustand zu erreichen;

(4) das gespülte Haar danach mit einem Fixiermittel mit einem Gehalt an einer wäßrigen Lösung einer schwach alkalischen Substanz, die fähig ist, die haarglättende Zusammensetzung chemisch zu neutralisieren, behandelt wird, wobei die wäßrige Lösung einen pH-Wert nicht größer als 9 aufweist;

(5) das neutralisierte Haar anschließend zur Entfernung des Fixiermittels mit Wasser gespült wird, worauf das Spülwasser wenigstens teilweise aus dem Haar entfernt wird, um einen zumindest handtuchtrockenen Zustand zu erreichen; und

(6) das Haar schließlich zur Stabilisierung seines Zustandes mit einem Stabilisierungsmittel mit einem Gehalt an einer wäßrigen Lösung eines wärmehärtbaren Aminopolyamid-Harzes behandelt wird.

2. Verfahren nach Anspruch 1, dadurch gekennzeichnet, daß die angewandte haarglättende Zusammensetzung ein inertes, gebildendes Verdickungsmittel in einer Menge enthält, daß der Wassergehalt der Zusammensetzung 50% des gesamten Gelvolumens überschreitet und die Zusammensetzung bei 25°C eine Viskosität zwischen 80 und 400 Ns/m² aufweist.

3. Verfahren nach Anspruch 1 oder 2, dadurch gekennzeichnet, daß der pH-Wert der wäßrigen haarglättenden Zusammensetzung innerhalb des schwach sauren Bereichs von 5,7 bis 7 liegt.

4. Verfahren nach einem der Ansprüche 1 bis 3, dadurch gekennzeichnet, daß das Harz in dem Stabiliserungsmittel aus einem kationischen wärmehärtbaren Aminopolyamid-Epichlorhydrin-Harz besteht.

5. Packung, geeignet zum Gebrauch in dem Verfahren gemäß Anspruch 1, dadurch gekennzeichnet, daß die in Anspruch 1 defnierte haarglättende Zusammensetzung, das in Anspruch 1 definierte Fixiermittel und das in Anspruch 1 definierte Stabilisierungsmittel getrennt voneinander, aber in der für die Verwendung in dem Verfahren erforderlichen Reihenfolge geordnet, in der Packung vorliegen.

6. Packung nach Anspruch 5, dadurch gekennzeichnet, daß sie eine wasserundurchlässige Kunststoffkappe zum Gebrauch in Verbindung mit der haarglättenden Zusammensetzung während der Wärmebehandlungsstufe des Verfahrens aufweist.

7. Packung nach Anspruch 5 oder 6, dadurch gekennzeichnet, daß die angewandte haarglättende Zusammensetzung ein inertes, gebildendes Verdickungsmittel in einer Menge enthält, daß der Wassergehalt der Zusammensetzung 50% des gesamten Gelvolumens überschreitet und die Zusammensetzung bei 25°C eine Viskosität zwischen 80 und 400 Ns/m² aufweist.

8. Packung nach einem der Ansprüche 5 bis 7, dadurch gekennzeichnet, daß der pH-Wert der wäßrigen haarglättenden Zusammensetzung innerhalb des schwach sauren Bereichs von 5,7 bis 7 liegt.

9. Packung nach einem der Ansprüche 5 bis 8, dadurch gekennzeichnet, daß das Harz in dem Stabilisierungsmittel aus einem kationische wärmehärtbaren Aminopolyamid-Epichlorhydrin-Harz besteht.

## Revendications

1. Un procédé pour détendre les boucles naturelles et les cheveux crépus de l'homme, par traitement de ceux-ci (y compris les parties de la racine du cuir chevelu) avec une composition pour détendre les boucles tout en manipulant les cheveux ainsi traités à la main et/ou en peignant de façont relativement droite et en rinçant ensuite les cheveux, caractérisé en ce que:

(1) la composition utilisée pour détendre les boucles est une solution aqueuse faiblement acide, présentant une valeur de pH inférieure à 7 mais supérieure à 5, d'un sel d'un acide capable de réduire les liaisons disulfures de la kératine dans les cheveux;

(2) les cheveux ainsi traités sont ensuite chauffées à une température supérieure à celle du corps humain, tandis que simultanément on maintient essentiellement constante la teneur en humidité des cheveux chauffés, jusqu'à ce qu'ils présentent un état détendu;

(3) le cuir chevelu et les cheveux ainsi détendus sont ensuite rincés à l'eau pour éliminer la composition pour détendre les boucles, et ensuite l'eau de rinçage est au moins partiellement éliminée des cheveux détendus pour arriver au moins à un état d'essorage par serviette;

(4) les cheveux rincés sont ensuite traités avec une composition de fixation comprenant une solution aqueuse, présentant un pH non supérieur à 9, d'une substance faiblement alcaline, capable de neutraliser chimiquement la composition pour détendre les boucles;

(5) les cheveux neutralisés sont ensuite rincés à l'eau pour éliminer la composition de fixation, l'eau de rinçage est ensuite au moins partiellement éliminée pour arriver au moins à un état d'essorage par la serviette; et

(6) les cheveux sont ensuite traités pour stabiliser leur état en y appliquant une composition stabilisante comprenant une solution aqueuse d'une résine thermodurcissable aminopolyamide.

2. Un procédé selon la revendication 1, dans lequel la composition utilisée pour détendre les boucles contient un épaississant inerte formateur de gel présent en quantité telle que le contenu en eau de la composition dépasse 50% de la masse totale du gel et que la composition présente une viscosité entre 80 et 400 Newton secondes par m2 à 25°C.

3. Un procédé selon la revendication 1 ou 2, dans lequel le pH de la composition aqueuse pour détendre les boucles se trouve dans le domaine faiblement acide de 5,7 à 7.

4. Un procédé selon l'une quelconque des revendications 1 à 3, dans lequel la résine de la composition stabilisante est une résine cationique thermodurcissable aminopolyamide-épichlorohydrine.

5. Un emballage approprié pour l'utilisation du procédé de la revendication 1, dans lequel la composition pour détendre les boucles définie à la revendication 1, la composition de fixation définie à la revendication 1 et la composition stabilisant définie à la revendication 1 sont chacune présentes séparément, mais disposées de façon à être utilisées les unes après les autres dans le susdit procédé.

6. Un emballage selon la revendication 5, qui comprend également un revêtement plastique

# 0011511

imperméable à l'eau destiné à être utilisé avec la composition pour détendre les boucles pendant l'étape du traitement de chauffage du procédé.

7. Un emballage selon la revendication 5 ou 6, dans lequel la composition pour détendre les boucles contient un épaississant inerte formateur de gel présent dans une quantité telle que le contenu en eau de la composition dépasse 50% de la masse totale du gel et la composition présente une viscosité entre 80 et 400 Newton secondes par m2 à 25°C.

8. Un emballage selon l'une quelconque des revendications 5 à 7, dans lequel le pH de la composition aqueuse pour détendre les boucles se trouve dans le domain faiblement acide de 5,7 à 7.

9. Un emballage selon l'une quelconque des revendications 5 à 8, dans lequel la résine dans la composition stabilisante est une résine cationique thermodurcissable aminopolyamide-épichloro-hydrine.